# EUROPEAN PATENT APPLICATION

(11) **EP 1 815 841 A1**
(43) Date of publication of application: **08.08.2007**
(21) Application number: 05027622.9
(22) Date of filing: 16.12.2005
(51) Int. Cl.: A61K 8/34, A61K 8/39, A61K 8/42, A61K 8/60, A61K 8/73, A61K 8/81, A61K 8/89, A61K 8/92, A61Q 5/12

(54) **Hair conditioning composition**

(71) Applicant: KPSS-Kao Professional Salon Services GmbH, 64297 Darmstadt (DE)
(72) Inventor: Bistram, Vera, 64683 Einhausen (DE); Grit, Mustafa, Dr., 64579 Gernsheim (DE)

(57) **Abstract**

The present invention relates to a gel type of hair conditioner composition showing excellent hair care properties especially for fine hair. Conditioning composition comprises at least one thickening polymer, and an emulsion which comprises at least one fatty alcohol, at least one emulsifier and at least one hair conditioning agent selected from non-ionic, anionic and oily substances. Conditioning composition are especially suitable for both rinse-off and leave-in usages for fine hair. The conditioner composition of the present invention further comprises active ingredients selected from hair restructuring ingredients, moisturising ingredients, sequestering agents and natural plant extracts showing hair conditioning and/or restructuring benefits.

## Description

The present invention relates to a gel type of hair conditioner composition showing excellent hair care properties, especially for fine hair.

Hair conditioners of various types either in application or in the form of preparation have found their wide range of usage in hair dressing practice. Among those rinse off types of conditioners have always been preferred in hair care. Usually rinse off type of conditioners is in the form of emulsions and comprising fatty alcohols and emulsifiers of different character as the main principal ingredients. In addition, they certainly comprise conditioning ingredients of various types and especially those of cationic surfactants and/or cationic polymers. A general overview on the known hair conditioning products and also their usual compositions can be found in the monography of K. Schrader, "Grundlage und Rezepturen der Kozmetika", 2nd Ed. 1989, pp 722 - 781.

Although the state of the art is quite advanced, some of the problems with conditioners are not either well addressed or solved. One of them is variability of conditioning properties of conventional conditioners depending on the hair type, i.e. damaged, fine, thick, normal, healthy hair types. As conditioners are used mainly for detangling and/or softening hair, the mainly used cationic compounds are deposited onto hair surface for achieving such effects. They usually cause weighing down effect (loss of volume by making hair heavy) although the surface conditioning properties are excellent. Without making a rule, conditioners are generally designed for different hair types depending on their damage levels. On the other hand, conditioners are highly desirable which are designed for different hair strengths depending on for example their diameter as defined such as coarse and fine hair. Although the big efforts, it has not been possible to offer a conditioner for fine hair without weighing down the hair which is at the same time suitable for both rinse off and leave-in usage.

Thus, the objective of the present application is to find out a conditioner composition, especially for fine hair, which does not show above mentioned disadvantageous and excellently suitable for using in rinse-off and leave-in applications.

It has surprisingly been found out that a gel type conditioner composition is excellently suitable for conditioning hair, especially for fine hair, in both rinse-off and leave-in usages, comprising a gel forming thickening polymer and/or mixture of polymers and comprising an emulsion, which comprises at least one fatty alcohol and at least one emulsifier as an emulsion base, and at least one hair conditioning agent selected form oily, anionic and non-ionic substances. The conditioner composition of the present invention is free of any cationic and amphoteric compounds as well as free of inorganic salts excluding compounds used for adjusting pH at relatively lower concentrations. Furthermore, the conditioners of the present invention can comprise active ingredients selected from, hair-restructuring, moisturising, sequestering compounds and natural plant extracts.

With the term rinse-off usage, it is meant that after application of conditioner onto hair and after a processing time of 1 to 30 min, preferably 1 to 15 min and more preferably 1 to 10 min, the hair is washed with water. With the term leave-in usage, it is meant that after application of the conditioner composition the hair is not washed with water but dried in air or by a hair drier. The preferred application of the conditioners of the present invention is rinse off.

The conditioners of the present invention make hair easy to comb and improve shine, elasticity and volume and body and in particular enhance structure of those damaged, chemically processed hair.

It should be well understood that the conditioner composition of the present invention is as well suitable for hair types other than fine hair. It has especially been found out that the conditioners are excellently suitable for fine-damaged hair with its excellent hair restructuring properties.

Anionic polymers such as acrylate types are usually used as thickeners in leave-in hair care products. They are certainly found as thickeners at relatively lower concentrations in especially cleansing compositions. When formulated in a rinse-off type of conditioning composition, after usage usually hair feeling is not acceptable as hair is difficult to comb through, does not feel soft, has almost no volume and body and not shiny at all. To the surprise of the inventors of the current invention, the conditioners as disclosed herein do not show those disadvantages, and, on contrary, are found to be very powerful in conditioning hair especially fine hair.

Polymers suitable for conditioners of the current inventions are, first of all, those anionic polymers of acrylate types. They are particularly preferred polymers showing thixotropic flow behaviour when dissolved or dispersed in aqueous medium for conditioner composition according to the present invention. Inclusion of thixotropic flow behaviour showing polymers gives conditioners of present invention attractive appearance and more importantly make application on hair easier. Those polymers of acrylate types are known with the trade names for example Carbopol from Goodrich, Pemulen from Noveon.

Another suitable polymer is a natural polymer xanthan gum, which shows as well thixotropic flow behaviour when dispersed or dissolved in water.

Conditioner compositions of the present invention can optionally contain non-ionic polymers such as hydroxyethylcellulose, hydroxypropylclellulose, xyloglucan, polyvinylalcohol, polyvinylpyrrolidone or their derivatives.

The concentration of polymers is typically from 0.01 to 5%, preferably 0.05 - 3%, and more preferably 0.1 - 2% by weight, most preferably 0.2 - 1.5% by weight calculated to the total composition.

Emulsion, which is part of the conditioner composition according to the invention, comprises at least one fatty alcohol or mixtures of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O from Cognis. The concentration of fatty alcohol(s) is in the range from 0.5 to 20%, preferably 0.5 to 15% and most preferably 1 to 10% by weight, calculated to total composition.

Conditioners of the present invention comprise one or more surfactants as emulsifier and/or solubilizers. Suitable surfactants are of anionic and non-ionic type or their mixtures. Suitable non-ionic surfactants are compounds from the category of alkyl polyglucosides with the general formula

R₁-0-(CH₂CH₂0)ₙ-Zₓ,

wherein R₁ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further non-ionic surfactants useful in the conditioner compositions according to invention are C₁₀-C₂₂-fafty alcohol ethoxylates. Especially suited C₁₀-C₂₂-fafty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 50, preferably about 10 and about 30.

Other additionally useful non-ionic surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Hydrogenated castor oil with variable ethylene glycol units is found to be suitable non-ionic solubilizers. Those are for example known from BASF under the trade name Cremophor.

Further optional nonionic surfactant components are fatty acid mono- and dialkanolamides, such as coco fatty acid monoethanolamide and myristic fatty acid monoisopropanolamide.

Suitable anionic surfactants are of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₂-(C₂H₄O)ₙ-O-CH₂COOX,

wherein R₂ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 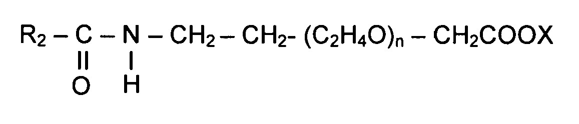 wherein R₂ and X have the above meanings, and n is in particular a number from 1 to 10, preferably 2.5 to 5. Such products have been known for some time and are on the market, for example, under the trade name "AKYPO^{®}" and "AKYPO-SOFT^{®}".

Among the surfactants, nonionic ethoxylated fatty alcohols are the most preferred ones for the compositions of the present invention. It is further preferred that the compositions comprise at least one nonionic surfactant and therefore anionic surfactant is always present together with at least one nonionic surfactant.

The concentration of surfactants is typically from 0.01 to 10%, preferably 0.1- 7.5%, and more preferably 0.5 - 5% by weight, calculated to the total composition. It is important that the surfactants used are chosen from those inorganic salt free ones as most of the commercially available liquid surfactants comprise inorganic salts. As mentioned above inorganic salts cause instability of the conditioner compositions of the present invention.

Conditioner compositions of the present invention do not comprise any cationic and amphoteric or zwitterionic surfactants.

Conditioner composition of the present invention comprise hair conditioning agents such as silicone oils either volatile or non-volatile, natural and synthetic oils. Among silicone oils those can be added to the conditioner composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Additional non-ionic conditioning agents are polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₃ CO (O CH₂ CH₂)ₙ OH formula I

R₃ CO (O CH₂ CH₂)ₙ O OC R₄ formula II

where R₃ and R₄ are independent from each other saturated, unsaturated or branched or non-branched alkyl chain with 7 to 21 C atoms and n is typically 2 - 100.

The conditioner composition can contain active ingredients selected from moisturisers, sequestering agents, hair restructuring agents and natural ingredients showing conditioning benefits.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

The sequestering agents are selected from polycarboxy acids. The preferred one is ethylene diaminetetraacetic acid, EDTA. Typical useful concentration range for sequestering agents is of 0.01 - 0.1 % by weight calculated to the total composition.

Hair restructuring agents preferred are ceramide type of compounds of the general formula 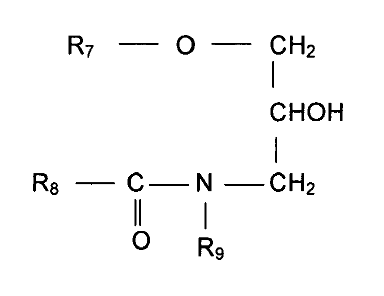 where R⁷ and R⁸ are independent from each other alkyl- or alkenyl group mit 10 to 22 carbon atoms and R⁹ is methyl, ethyl, n-propyl or isopropyl or a hydroxyl alkyl with 1 to 3 C atoms. Cetyl - PG - hydroxyethyl palmitamide is the most preferred compound of the above structure.

Another preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the conditioners of the present invention can be in the range of 0.01 to 2 and especially 0.01 to 1% by weight calculated to the total weight of the composition. The fatty acids may be contained at a level of 0.01 to 2.5% and especially 0.01 to 1 % by weight calculated to the total weight of the composition. Phytosterol concentration of the conditioners is less than 1% and preferably less than 0.5% by weight calculated to the total weight of the composition. It should be noted that the combination of those three ingredients is especially preferable for achieving the optimum benefits from the conditioners of the present invention.

Natural plant extracts showing hair conditioning and/or restructuring effects can be used in the conditioners. Those are preferably the extracts from almond, Aloe Vera, coconut, mango, peach, lemon, wheat, rosemary, apricot, algae, grapefruit, sandalwood, lime and orange. Those extracts used are the ones commercially available and generally include organic solvents such as propylene glycol, butylenes glycol, ethanol, isopropanol. The active matter in those extracts can vary largely, i.e. in the range of 1 - 30% by weight. Concentration of those as an extract depending on the compatibility with the other ingredients can be in the range of 0.1 to 5%, preferably 0.1 to 2% by weight calculated to the total composition.

The pH of the conditioners of the present invention varies from 4 to 8, particularly 5 to 7.5 and more particularly 5 to 7.

The pH of the conditioner composition is adjusted to the required pH by using alkaline solution such as sodium hydroxide, potassium hydroxide, triethanolamine.

For adjusting the pH of the conditioner compositions, following ingredients can as well be used: Organic acids such as citric acid, lactic acid, tartaric acid, malic acid, maleic acid, fumaric acid, levulinic acid, butyric acid and hydroxybutyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that conditioner composition is so obtained has a pH value between 4 and 8.

The conditioner compositions may contain organic solvents, for example, as penetration enhancer such as ethanol propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol. Concentration of organic solvents in the conditioner composition can be in the range from 0.1 to 20% by weight, preferably 0.5 to 15% by weight, and more preferably 1 to 15% by weight calculated to the total composition. The organic solvents may at the same time serve to solubilize ingredients, which are not readily soluble or dispersible in the conditioner composition.

Conditioner composition of the present invention comprises preferably one or more oil soluble UV filter. Without limiting the scope the preferred are Benzophenone-3 and octylmethoxycinnamate at a concentration of 0.01 to 3% by weight calculated to total composition.

The conditioner compositions can have viscosity values between 1,000 mPa.s to 100,000 mPa.s, preferably 1,000 mPa.s to 80,000 mPa.s, more preferably 5,000 mPa.s to 60,000 mPa.s and more preferably 10,000 mPa.s to 40,000 mPa.s measured at 20°C with Brookfield viscosimeter with, for example, Spindle 4 at 10 rpm. The viscosity values are read after 60 seconds from the start of the measurement. In the selection of the viscosity, special attention must be paid to the way of application and packaging to be used.

The following examples are to illustrate the invention, but not limiting it.

### Example 1

| | |
|---|---|
| Carbopol 2020 | 0.85 |
| Sodium hydroxide | q.s. to pH 6.0 |
| Panthenol | 0.50 |
| Dimethicone | 0.20 |
| Ctearyl alcohol | 3.00 |
| Ceteareth 20 | 1.00 |
| Ceramide of the formula* | 0.20 |
| Behenic acid | 0.20 |
| Avocadin | 0.50 |
| Benzophenone-3 | 0.30 |
| Fragrance | q.s. |
| Preservative | q.s. |
| Water | to 100 |

| | |
|---|---|
| * as given in the description R₇ is C₁₅H₃₁; R₈ is C₁₆H₃₃ and R₁₅ is hydroxyethyl. | |

Carbopol 2020 is dispersed/dissolve in water and neutralised by addition of sodium hydroxide. In a separate vessel, emulsion is prepared by melting first fatty alcohol, ceteareth 20, ceramide, behenic acid and avocadin. Subsequently dimethicone (Silicone oil AK 500) is added and the mixture is homogenized. After cooling down to around 40°C, it is combined with the polymer solution/dispersion and preservative and fragrance is added. The pH of the composition is 6.0 and the viscosity is 55,000 mPa.s as measure in the same way as given above.

The composition is tested in a half-side comparative test first of all against no product in leave in and rinse off usage. The product is applied onto hair after shampooing with a commercially available shampoo and towel drying the hair. The test persons were selected form women having fine and shoulder length hair. In both tests, it is obviously observed that the composition improves hair combability and shine and as well gives hair elasticity, volume and body, and shine.

In another half side comparative test the composition of example 1 is tested against a conventional conditioner composition with the following content.

### Example 2 (comparative formula, not inventive)

| | |
|---|---|
| Panthenol | 0.50 |
| Dimethicone | 0.20 |
| Ctearyl alcohol | 5.00 |
| Cetrimonium chloride | 1.00 |
| Fragrance | q.s. |
| Preservative | q.s. |
| Citric acid/sodium hydroxide | q.s. to pH 4.5 |
| Water | to 100 |

Both compositions (inventive and comparative) were tested against each other in a half-head test on 10 women having fine and shoulder length hair. For this purpose whole head of test person was washed with a commercially available shampoo and the equal amount from both conditioners were applied to each side and processed for 5 min. Subsequently rinsed off and towel dried and further dried with a dryer. In wet and dry stage sensory evaluations were made on the hair properties given in Table II.

The results of the test is summarised in the tables I and II for rinse off and leave in usages.

**Table I: Half side test results - Rinse off usage**

| Prefered | | | | |
|---|---|---|---|---|
| | Parameter | Example 1 | Example 2 | No preference* |
| Wet | Easy combing | 3 | 3 | 4 |
| | Smoothness | 4 | 4 | 2 |
| | Roughness | 1 | 1 | 8 |
| Dry | Easy combing | 3 | 4 | 3 |
| | Smoothness | 4 | 3 | 3 |
| | Elasticity | 6 | 2 | 2 |
| | Volume | 7 | 1 | 2 |
| | Shine | 6 | 1 | 3 |

**Table II: Half side test results - Leave-in usage**

| Prefered | | | | |
|---|---|---|---|---|
| | Parameter | Example 1 | Example 2 | No preference* |
| Wet | Easy combing | 3 | 2 | 5 |
| | Smoothness | 2 | 2 | 6 |
| | Roughness | 1 | 2 | 7 |
| Dry | Easy combing | 4 | 2 | 4 |
| | Smoothness | 5 | 2 | 3 |
| | Elasticity | 8 | 1 | 1 |
| | Volume | 8 | 1 | 1 |
| | Shine | 7 | 1 | 1 |

| | | | | |
|---|---|---|---|---|
| *: with the term "no preference" it is meant that both sides are evaluated to be the same. | | | | |

Similar results are obtained with the following inventive examples.

### Example 3

### Leave-in conditioner

| | |
|---|---|
| Carbopol 2020 | 0.30 |
| Sodium hydroxide | q.s. to pH 6.0 |
| Dimethicone | 0.10 |
| Glycerin | 0.50 |
| Ethanol | 8.00 |
| Ctearyl alcohol | 2.00 |
| Ceteareth 20 | 1.00 |
| Ceramide of the formula* | 0.10 |
| Behenic acid | 0.10 |
| Avocadin | 0.20 |
| Almond oil | 0.10 |
| Fragrance | q.s. |
| Preservative | q.s. |
| Water | to 100 |

| | |
|---|---|
| * as given in the description R₇ is C₁₅H₃₁; R₈ is C₁₆H₃₃ and R₁₅ is hydroxyethyl. | |

Formulation is prepared as described for example 1.

In the leave in and rinse off usage similar results are observed as in the case of the example 1. Users with fine hair especially prefer the composition.

## Claims

1. Conditioning composition for hair suitable for both rinse-off and leave-in usages **characterised in that** it comprises
a- at least one thickening polymer, and
b- an emulsion which comprises at least one fatty alcohol, at least one emulsifier and at least one hair conditioning agent selected from non-ionic, anionic and oily substances, and
has a pH between 4 and 8,
with the condition that the composition is free of any cationic and amphoteric compounds and of inorganic salts.

2. Conditioning composition according to claim 1 **characterised in that** it comprises as a thickening polymer an acrylate type polymer and/or xanthan gum or their mixture at a concentration 0.01 to 5% by weight calculated to the total composition.

3. Conditioning composition according to claims 1 and 2 **characterised in that** it comprises as emulsifier surfactants selected from anionic and non-ionic types and at a concentration of 0.1 to 10% by weight calculated to the total composition.

4. Conditioning composition according to claim 3 **characterised in that** it comprises as emulsifier at least one ethoxylated fatty alcohol.

5. Conditioning composition according to any of the preceding claims **characterised in that** it further comprises active ingredients selected from hair restructuring ingredients, moisturising ingredients, sequestering agents and natural plant extracts.

6. Conditioning composition according to claim 5 **characterised in that** it comprises as hair restructuring agents ceramide according to the formula 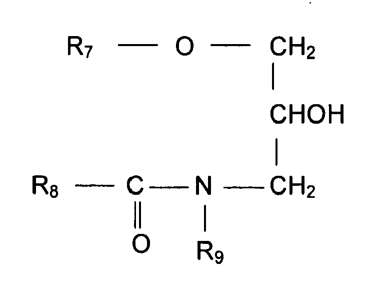 where R⁷ and R⁸ are independent from each other alkyl- or alkenyl group mit 10 to 22 carbon atoms, R⁹ is methyl, ethyl, n-propyl or isopropyl or hydroxyl alkly with 1 to 4 C atoms and/or fatty acids with 10 to 24 carbon atoms and/or phytosterol and/or their mixtures.

7. Conditioning composition according to any of the preceding claims **characterised in that** it comprises organic solvents.

8. Conditioning composition according to any of the preceding claims **characterised in that** it comprises oil soluble UV filter.

9. Use of conditioning composition according to any of the preceding claims for conditioning fine hair.
